# EUROPÄISCHE PATENTSCHRIFT

(11) **EP 2 104 471 B1**
(45) Veröffentlichungstag und Bekanntmachung des Hinweises auf die Patenterteilung: **25.01.2017**
(21) Anmeldenummer: 07845635.7
(22) Anmeldetag: 17.12.2007
(51) Int. Cl.: A61F 2/30, A61L 27/02

(54) **DENTALIMPLANTAT UND VERFAHREN ZU DESSEN HERSTELLUNG**
DENTAL IMPLANT AND METHOD FOR THE PRODUCTION THEREOF
IMPLANT DENTAIRE ET PROCÉDÉ DE FABRICATION DE CELUI-CI

(30) Priorität: 22.12.2006 CH 21022006
(43) Veröffentlichungstag der Anmeldung: 30.09.2009
(73) Patentinhaber: THOMMEN MEDICAL AG, 2540 Grenchen (CH)
(72) Erfinder: SCHLOTTIG, Falko, 4414 Füllinsdorf (CH); ORTEGA CRUZ, Luis Alfonso, 2545 Selzach (DE)
(74) Vertreter: Bremi, Tobias Hans
(86) Internationale Anmeldenummer: PCT/CH2007/000638
(87) Internationale Veröffentlichungsnummer: WO 2008/077263

(56) Entgegenhaltungen:
- EP-A- 1 825 830
- WO-A-01/19556
- WO-A-01/72664
- WO-A-98/43927
- WO-A-03/045268
- WO-A-03/059407
- WO-A-2007/090529
- WO-A-2008/009272
- DD-A1- 129 195
- DE-A1- 10 243 101
- DE-A1- 19 638 927
- DE-A1-102005 005 656
- DE-A1-102006 036 039
- DE-C1- 19 726 961
- DE-U1-202004 020 338

## Beschreibung

### TECHNISCHES GEBIET

Die vorliegende Erfindung betrifft ein Implantat, insbesondere ein Dentalimplantat, mit einer porösen Oberfläche zum wenigstens teilweisen Einsetzen in einen Knochen, welches verbesserte Osteointegrationseigenschaften aufweist. Das Implantat ist dabei keramisch, kann aber auch metallisch sein. Weiterhin betrifft die vorliegende Erfindung ein Verfahren zur Herstellung eines solchen Implantates sowie Verwendungen eines solchen Implantates.

### STAND DER TECHNIK

Verletzte oder beschädigte Teile des Hart- und/oder Weichgewebes des menschlichen Körpers werden am Besten wiederhergestellt, indem körpereigenes Hart- und/oder Weichgewebe verwendet wird. Dies ist aus verschiedenen Gründen nicht immer möglich, und daher kommt in vielen Fällen synthetisches Material als temporäres (bioabbaubares respektive postoperativ entfernbares) oder permanentes Ersatzmaterial zum Einsatz.

Implantate, welche im Hart- und/oder Weichgewebe verankert werden, dienen dem temporären oder permanenten Ersatz oder dem Support von unfall-, abnützungs-, mangelerscheinungs- oder krankheitsgeschädigten oder sonst degenerierten Teilen des Bewegungsapparates, einschliesslich insbesondere des Kauapparates. Als Implantat wird normalerweise ein künstliches, chemisch stabiles Material bezeichnet, welches als plastischer Ersatz oder zur mechanischen Verstärkung in den Körper eingebracht wird (vgl. z.B. Roche Lexikon Medizin, Urban & Fischer, (Hrsg.); 5. Aufl. 2003). Die Hilfs- und Ersatzfunktion im Körper wird auf Basis der mechanischen Eigenschaften und des Implantatdesigns übernommen. So sind beispielsweise Hüft- und Kniegelenksprothesen, Wirbelsäulenimplantate und Dentalimplantate seit vielen Jahren im erfolgreichen klinischen Einsatz.

Für die Implantatverankerung und die Implantatverträglichkeit an der Grenzfläche Implantatoberfläche / angrenzendes Gewebe hat die Implantatoberfläche eine grosse Bedeutung. So haben Messungen gezeigt, dass Implantate mit glatter Oberfläche beinahe unabhängig vom verwendeten Basismaterial nur wenig im Knochen verankert werden (schlechte Osteointegration), während Implantate mit einer strukturierten Oberfläche eine gute mechanische und bei entsprechender Gestaltung der Oberfläche auch eine gute biologische Verbindung mit dem umgebenden Hart- oder Weichgewebe eingehen (vgl. z.B. Titanium in Medicine, Material Science, Surface Science, Engineering, Biological Responses and Medical Applications Series: Engineering Materials, Brunette, D.M.; Tengvall, P.; Textor, M.; Thomsen, P. (Eds.)).

Die für ein genügendes Einwachsen benötigte Zeit, eine für Implantate wichtige und zentrale Eigenschaft, wird als Osteointegrationszeit, respektive im Dentalbereich auch als Osseointegrationszeit bezeichnet. Damit wird die Zeit beschrieben, die vergeht, bis sich die Knochensubstanz in genügender Stärke und dauerhaft mit der Implantatoberfläche verbunden hat, das heisst sich gewissermassen in diese integriert hat.

Zur Oberflächenbehandlung und Oberflächenstrukturierung werden verschiedenste Methoden verwendet, siehe z.B. A Guide to Metal and Plastic Finishing (Maroney, Marion L.; 1991); Handbook of Semiconductor Electrodeposition (Applied Physics, 5) (Pandey, R. K., et. al.; 1996); Surface Finishing Systems: Metal and Non-Metal Finishing Handbook-Guide (Rudzki, George J.; 1984); Titanium in Medicine, Material Science, Surface Science, Engineering, Biological Responses and Medical Applications Series: Engineering Materials, (Brunette, D.M.; Tengvall, P.; Textor, M.; Thomsen, P. (Eds.)) und Materials and Processes for Surface and Interface Engineering (NATO Asi Series. Series E, Applied Sciences, 115, Pauleau, Ives (Editor); 1995); und die darin genannten Referenzen.

Implantate werden heute aus verschiedensten Materialien hergestellt, wie beispielsweise aus Titan, Niob, Zirkon, Tantal, aus Legierungen wie z.B. Titanlegierungen, Implantatstahl, aus CoCr Legierungen, aus verschiedenen Polymeren und Keramiken z.B. auf Basis von Zirkonoxiden, Aluminiumoxiden, Titanoxiden etc..

Neben den mechanischen Bearbeitungsverfahren können Implantate beispielsweise auch durch eine Kombination aus Giessen und Sintern hergestellt werden. Diese Verfahren sind für Metall als MIM (Metal Powder Injection Molding) und für Keramik als CIM (Ceramic Injection Molding) bekannt, so z.B. aus der US 2004/0038180.

Zur Herstellung von Zahnimplantaten können beide Verfahren auch gekoppelt werden, wie in EP 1 570 804 A1 beschrieben wird. Weiterhin ist eine Kombination mit mechanischen Bearbeitung des durch MIM oder CIM hergestellten Implantates möglich so beispielsweise in EP 1 570 804 A1 geschrieben, dass die Oberfläche nach den Sintern entweder durch Strahlbehandlung oder durch chemische Oberflächenmodifikation (z.B. Säureätzen) nachbehandelt werden kann.

Für viele Implantate, speziell für Zahnimplantate, werden hauptsächlich Titan und seine Legierungen verwendet, da diese Materialien ein ausreichend niedriges Elastizitätsmodul und eine relativ hohe Festigkeit aufweisen. Allerdings haben Messungen gezeigt, dass sich Titan- Implantate mit glatter Oberflächenstruktur nur ungenügend im Knochen verankern, während Implantate mit aufgerauter Oberfläche einen bezüglich der Zug- und Torsionsfestigkeit merklich verbesserten Knochen-Implantat-Verbund ergeben.

In der EP 0 388 576 A1 wird deshalb vorgeschlagen, auf einer metallischen Implantatoberfläche in einem ersten Schritt mittels Sandstrahlen eine Makrorauhigkeit aufzubringen und diese anschliessend mittels Behandlung in einem Säurebad mit einer Mikrorauhigkeit zu überlagern. So kann die Implantatoberfläche mittels Sandstrahlen aufgeraut und anschliessend mit einem Ätzmittel, z. B. Fluorwasserstoffsäure oder einem Chlorwasserstoffsäure/Schwefelsäuregemisch behandelt werden. Durch diese Strukturierung der Oberfläche wird eine sichere Verbindung zwischen Hartgewebe und Metall erreicht.

Im Bereich der Dentalimplanate ist Titan, insbesondere im vorderen, sichtbaren Oralbereich, aus ästhetischen Gründen ungeeignet, da sich das Material optisch von der Hart- und der sichtbaren Weichgewebeumgebung unterscheidet. Daher ist es erstrebenswert, einen anderen Werkstoff, der diese Nachteile nicht aufweist, einzusetzen. Mit keramischen Werkstoffen wie beispielsweise Zirkonoxid, Titanoxid oder Aluminiumoxid oder Mischungen hiervon stehen Materialien zur Verfügung, die eine äusserst hohe Festigkeit aufweisen, insbesondere, wenn die Formkörper heissisostatisch gepresst oder heiss-isostatisch nachverdichtet sind. Eine spezifische yttriumstabilisierte Zirkonoxidkeramik, die etwa 92,1-93, 5 Gew.-% Zr02, 4,5-5, 5 25 Gew.-% Y203 und 3,8-2, 2 Gew.-% HfO2 aufweist, ist beispielsweise aus der US 6,165,925 bekannt. Andere gängige Keramiken werden in der Einleitung der US 6,165,925 diskutiert.

Der Einsatz von Keramik, beispielsweise einer Zirkonoxidkeramik, einer Titanoxidkeramik oder einer Aluminiumoxidkeramik, als Material zur Herstellung eines im Hart- oder Weichgewebe verankerten Implantates ist aufwändig, da es für eine ausreichende mechanische Stabilität der Keramik notwendig ist, diese ohne messbare Porosität herzustellen, wobei sich i.d.R. gleichzeitig eine glatte, äusserst harte Oberfläche ergibt.

Für glatte Keramikoberflächen ist kein direkter und ausreichend mechanisch stabiler Verbund mit dem umgebenden Hartgewebe zu erwarten. Daher werden Implantate aus reinen Keramiken wie Zirkonoxid, Titanoxid oder Aluminiumoxid oder Mischungen hiervon bisher kaum im direkten Kontakt zu Hartgewebe verwendet. Zur Verankerung im Hartgewebe werden konstruktive Verbunde mit metallischen Implantatmaterialien eingesetzt, beispielsweise in der Hüftendoprothetik oder in der oralen Implantologie.

Beispielsweise wird in der DE 195 30 981 A1 eine präfabrizierte vollkeramische Implantatkonstruktion aus Zirkondioxid zum zahnfarbenen Aufbau implantatgetragener artifizieller Kronenstümpfe beschrieben. Das eigentliche Implantat besteht dabei aus oberflächenstrukturiertem metallischem Titan, die Ästhetik des sichtbaren Teiles wird über eine Zirkonoxidkeramik dargestellt.

In der WO 2004/096075 A1 wird ein Zahnimplantat aus einem einteiligen Grundkörper beschrieben, der aus Zirkonoxid oder aus einer Zirkonoxid/Aluminium Mischung besteht. Eine Oberflächenbehandlung ist nicht beschrieben, und es ist fraglich, ob eine solche Implantatstruktur überhaupt genügende Osseointegration zeigt.

Die FR 2 721 196 A1 beschreibt ein einteiliges, auf Zirkoniumoxid basierendes Implantat. Zur Verbesserung der Osteointegration soll der entsprechende Implantatteil mit einer Beschichtung, beispielsweise aus Hydroxyapatit, versehen werden.

In der WO03/045268 A1 wird ein Keramikimplantat auf der Basis von Zirkonoxid beschrieben. Die Aussenfläche des Verankerungsteils wird zumindest teilweise entweder mit einem abtragenden Verfahren aufgerauht oder mikrostrukturiert oder mit einer Beschichtung versehen. Dabei werden nach einer Strahlbehandlung, etwa durch Sandstrahlen, auch chemische Verfahren, insbesondere Ätzverfahren in Betracht gezogen, die teilweise ergänzend als Nachbehandlung zu einer vorherigen mechanischen Behandlung zur Anwendung kommen können. Bevorzugt ist insbesondere zunächst eine Strahlbehandlung, etwa durch Sandstrahlen mit Al203, und anschliessend eine Ätzbehandlung mit Phosphorsäure, Schwefelsäure, Salzsäure oder Mischungen hiervon. Weiterhin kann das behandelte Implantat in einer geeigneten Flüssigkeit, beispielsweise deionisiertem Wasser, oder in einer NaCl-Lösung gelagert werden. Auf diese Weise wird vermieden, dass die Oberfläche vor dem Einsetzen des Dentalimplantats durch Bestandteile der Luft ihre Aktivierung ganz oder teilweise verliert. So wird eine Osteointegration unterstützt.

Die Problematik dabei ist, dass mit einer solchen kombinierten Behandlung die Rautiefe infolge der hohen Härte der Zirkonoxidkeramik gering bleibt und dass die Keramik gegenüber der Behandlung mit Phosphorsäure, Schwefelsäure, Salzsäure oder Mischungen hiervon chemisch äusserst stabil ist.

In der DE 10 2005 013 200 wird ein zweiteiliges keramisches Implantat beschrieben inklusive einer Mikro- und Makrostrukturierung und der chemischen respektive biochemischen/ pharmazeutischen Modifizierung der Oberflächen bzw. ausgewählter Oberflächen des Implantates. Ein Verfahren zur Erreichung dieser Oberflächenstruktur oder der Oberflächenmodifizierung wird nicht spezifisch angegeben.

### DARSTELLUNG DER ERFINDUNG

Der Erfindung liegt somit u.a. das Ziel zugrunde, die Nachteile des Standes der Technik zu vermeiden und Implantate vorzuschlagen, welche im Hart- und Weichgewebe zügig und nachhaltig verankern und somit gute Osteointegration resp. Osseointegration zeigen. Konkret geht es also darum, ein verbessertes metallisches und/oder keramisches Implantat mit einer strukturierten, insbesondere porösen Oberfläche zum wenigstens teilweisen Einsetzen in Hartgewebe wie in einen Knochen und/oder in Weichgewebe vorzuschlagen. Ferner soll ein geeignetes Herstellverfahren hierfür angegeben werden.

Bevorzugtermassen geht es dabei um ein Dentalimplantat. Das Herstellungsverfahren ist also insbesondere bevorzugt ein Herstellungsverfahren zur Herstellung eines Dentalimplantates.

Gleichermassen geht es aber auch um Implantate ausserhalb des Bereichs der Dentalimplantate. Das Herstellungsverfahren ist also alternativ ein Herstellungsverfahren für Implantate ausserhalb des Dentalimplantat-Bereiches.

Insbesondere geht es also um ein Verfahren zur Herstellung eines metallischen und/oder keramischen Implantates mit einer strukturierten, insbesondere porösen Oberfläche zum wenigstens teilweisen Einsetzen in Hartgewebe wie in einen Knochen und/oder in Weichgewebe, wobei das Implantat wenigstens bereichsweise unter Zuhilfenahme eines kaltisostatischen Pressens, Giessens und/oder Spritzens (CIM, MIM) zu einem Grünling mit anschliessendem Sintern zum Implantat hergestellt wird. Das Verfahren ist dabei dadurch gekennzeichnet, dass die Oberfläche vor dem Sintern derart verändert und/oder vorbereitet wird, dass nach dem Sintern ohne zusätzliche Nachbearbeitung eine makroporöse Oberfläche vorliegt. Dies schliesst aber eine zusätzliche Nachbearbeitung nicht aus, sofern sie noch sinnvoll oder erforderlich ist, beispielsweise kann es sinnvoll sein, eine insbesondere bevorzugtermassen chemische Nachbearbeitung zur Erzeugung einer Mikroporosität anschliessen zu lassen.

Unter einer makroporösen Oberfläche wird dabei verstanden, dass eine Topographie (topologische Strukturierung) und/oder Poren mit einer mittleren Grösse von mehr als 2µm, vorzugsweise mehr als 5µm, ganz vorzugsweise > 20 µm vorhanden ist/sind.

Bei der Herstellung eines Implantats durch eine Kombination aus Giessen und Sintern bzw. durch MIM oder CIM oder einer Kombination beider Verfahren sind bisher keine Möglichkeiten bekannt, innerhalb dieses Verfahrens zur Zahnimplantatherstellung eine geeignete Rauhigkeit respektive Porosität an der Oberfläche des Implantates anzubringen, im Stand der Technik finden sich nur Verfahren, in welchen die Modifikation der Oberfläche in einem an das Sintern anschliessenden Schritt erfolgt.

In Bezug auf die beiden Verfahren CIM und MIM sei beispielhaft verwiesen auf die WO 97/38811 sowie auf die US 5,482,671, deren Offenbarungsgehalt bezüglich dieser beider Verfahren ausdrücklich in die vorliegende Offenbarung eingeschlossen wird.

Grundsätzlich wird bei diesem Herstellungsverfahren so vorgegangen, dass zunächst ein Pulver als Ausgangsmaterial, beispielsweise als Mischung, vorgelegt wird. Anschliessend findet das kaltisostatische Pressen, Giessen und/oder Spritzen statt, gefolgt von einem Sinterungsvorgang, bei welchem die eigentliche Keramik respektive der eigentliche stabile Metallverbund gebildet wird. Zur Herstellung werden also aus künstlich hergestellten Rohstoffen zunächst sogenannte Grünlinge oder Grünkörper geformt. Diese Grünkörper enthalten neben den keramischen oder metallischen Pulvermischungen normalerweise auch noch Feuchte und organische Bindemittel. Zunächst wird der Grünkörper getrocknet. Dann müssen i.d.R. alle, bei hohen Temperaturen flüchtigen, verdampfenden oder verbrennenden Anteile insbesondere des Binders im sogenannten Ausbrennprozess (auch als Entkohlung oder Entbinderung bekannt) aus dem keramischen Grünling entfernt werden. Nach dem Trocknen und Ausbrennen bzw. Entbinderung/Verkoken wird das Gefüge des Grünlings lediglich durch Adhäsionskräfte zusammengehalten und bedarf einer besonders sorgfältigen Handhabung während der weiteren Prozessschritte. Zuletzt findet das Brennen oder Sintern der Keramik statt. In diesem Schritt erhält der keramische Körper seine Festigkeit.

Gemäss einer ersten bevorzugten Ausführungsform ist das Verfahren dadurch gekennzeichnet, dass der Grünling nach dem kaltisostatischen Pressen, Giessen und/oder Spritzen und vor dem endgültigen Sintern durch Strahlen der Oberfläche des Grünlings modifiziert wird.

Gemäss der Erfindung ist das Verfahren dadurch gekennzeichnet, dass als Strahlmittel für das Strahlen ein abrasives und/oder oberflächenverdichtendes Strahlmittel verwendet wird. Insbesondere bevorzugt verwendet man ein metallisches Strahlmittel wie Stahlkugeln, ein keramisches Strahlmittel wie Al₂O₃, ZrO₂, SiO₂, Ca Phosphate, TiO₂, NaO₂, CaO, MgO, ein organisches oder natürliches Strahlmittel wie Nussschalen oder Reis in verschiedenen Korn- und Splittergrössen, oder Mischungen der genannten Strahlmittel. Alternativ oder zusätzlich kann es sich beim Strahlmittel für das Strahlen um Eiskugeln oder Eispartikel handeln, organische Strahlmittel wie Stearate, Wachse, Paraffine oder bevorzugt Carbamid, Melaminharz, Biuret, Melamin, Ammoniumkarbonat und Ammoniumbikarbonat oder Mischungen davon.

Bevorzugtermassen werden Strahlmittel verwendet, welche vor dem abschliessenden Sintern rückstandsfrei bei Temperaturen bis max. 600°C oder max. 300°C entfernt werden können, wobei diese Entfernung bevorzugtermassen in einer oxidierenden oder reduzierenden oder inerten Atmosphäre durchgeführt wird, wie insbesondere bevorzugt unter O₂, N₂, NH₄, Ar oder im Vakuum. Ein in dieser Hinsicht bevorzugtes Strahlmittel ist Ammoniumbikarbonat, welches schon 65°C von der Oberfläche des Grünlings sublimiert und die gewünschte Struktur in der Oberfläche hinterlässt.

Typischerweise liegt die Korngrösse des Strahlmittels in den Bereichen 0.01 - 0.25 mm bevorzugt im Bereich von 10 - 200 µm, insbesondere bevorzugt im Bereich von 50 - 110 µm. Bevorzugtermassen wird ein Strahldruck im Bereich von 0.2 - 7 bar, bevorzugtermassen zwischen 0.2-5 bar, insbesondere bevorzugt im Bereich von 0.8 bar gewählt. Typischerweise wird die Strahlbehandlung während einer Zeitdauer zwischen 15 und 65 Sekunden, die bevorzugt zwischen 35 und 55 Sekunden, insbesondere bevorzugt im Bereich von 50 Sekunden durchgeführt. Es erweist sich dabei als vorteilhaft, wenn der Abstand von der Düse zum Implantat im Bereich von 25-80 mm, besondere zwischen 25 und 60 mm, insbesondere bevorzugt im Bereich von 30 mm gewählt wird. Generell vorteilhaft ist es, einen Bohrungsdurchmesser der Düse im Bereich von 0.8 -1.2mm, bevorzugtermassen 0.8-1.0mm zu wählen. Ganz besonders bevorzugt ist die Verwendung einer Flachdüse, d.h. eine Düse, deren Austrittsöffnung im Querschnitt nicht kreisförmig sondern länglich (rechteckig, mit oder ohne gerundete Kanten, oval, quasi-oval) ist. Die Breite der Düsenöffnung ist dabei vorzugsweise wenigstens 0.2 mal grösser als die Höhe, möglich ist z.B. eine Breite im Bereich von 1.2 - 1.4 mm und eine Höhe im Bereich von 0.6 - 1.0 vorzugsweise 0.8mm.

Gemäss der Erfindung wird als Strahlmittel eine Mischung aus zwei Strahlmitteln mit unterschiedlicher Korngrösse eingesetzt. Auf diese Weise kann unter anderem eine gewissermassen bimodale Verteilung der erzeugten Rauigkeit gewährleistet werden, das heisst es ergeben sich feine sowie auch grobe Strukturen. Unterschiedliche Strahlmittel heisst in diesem Zusammenhang unterschiedlich hinsichtlich der mittleren Korngrösse, nicht notwendigerweise aber hinsichtlich des Materials des Strahlmittels. Es ist beispielsweise möglich, als Strahlmittel eine Mischung aus einem ersten Anteil mit einer groben Verteilung der Korngrösse und einem zweiten Anteil mit einer feinen mittleren Korngrösse aus dem gleichen Material zu verwenden (ausdrücklich bimodale Verteilung eines einzigen Materials).

Bevorzugt wird aber zusätzlich zur unterschiedlichen mittleren Korngrösse ein unterschiedliches Material, so beispielsweise ein organisches grobes Strahlmittel und ein anorganisches feines Strahlmittel.

Nach der Erfindung liegt der Unterschied in der mittleren Korngrösse der unterschiedlichen Strahlmittel in der Mischung im Bereich von einem Faktor 5-10.

So kann beispielsweise ein erstes Strahlmittel in der Mischung vorhanden sein, welches eine mittlere Korngrösse im Bereich von 0.1-0.2 mm, bevorzugt im Bereich von 0.2-0.8 mm aufweist. Bevorzugtermassen handelt es sich dabei um ein organisches Strahlmittel, beispielsweise aus Obstkernen (beispielsweise Pfirsichkerne und/oder Aprikosenkerne).

Des weiteren kann ein zweites Strahlmittel in der Mischung vorhanden sein, welches eine mittlere Korngrösse im Bereich von 0.01-0.1 mm, bevorzugt im Bereich von 0.03-0.9 mm aufweist, wobei es sich dabei bevorzugt um ein anorganisches Strahlmittel, insbesondere auf Basis von Aluminiumoxid (Al2O3), handelt.

Generell liegt bevorzugtermassen das Verhältnis von erstem zu zweitem Strahlmittel im Bereich von 5:1 - 1:5, bevorzugt im Bereich von 3:1 - 1:1.

Weiterhin ist es besonders bevorzugt, dass in wenigstens zwei Schritten gestrahlt wird, wobei in einem Schritt die genannte Mischung verwendet wird und in einem anfolgenden Schritt nur noch das Strahlmittel mit der geringeren Korngrösse, bevorzugt als ein anorganisches Strahlmittel, eingesetzt wird, wobei bevorzugtermassen der zweite Schritt unter einem wenigstens 5-10 mal kleineren Strahldruck gefahren wird.

Bevorzugtermassen wird beim Schritt unter Verwendung einer Mischung ein Strahldruck im Bereich von 2-7 bar, bevorzugt 3-5 bar verwendet. Des weiteren kann bei diesem Schritt die Zeit der Behandlung im Bereich von 15-65 Sekunden, bevorzugt im Bereich von 25-45 Sekunden liegen. Des weiteren kann der Abstand von der Düse zum Implantat im Bereich von 25-80 mm liegen. Des weiteren kann der Bohrungsdurchmesser der Düse im Bereich von 1.2-2.0 mm liegen.

Bevorzugtermassen kann bei einem gegebenenfalls vorhandenen zweiten Schritt ein Strahldruck im Bereich von 0.2-0.8 bar, bevorzugt im Bereich von 0.2-0.4 bar verwendet werden. Des weiteren kann die Zeit der Behandlung im Bereich von 10-35 Sekunden, bevorzugt im Bereich von 15-25 Sekunden liegen. Des weiteren kann der Abstand von der Düse zum Implantat im Bereich von 30-50 mm liegen. Des weiteren kann der Bohrungsdurchmesser der Düse im Bereich von 0.8 mm-1.2 mm liegen.

Wie bereits erläutert ist es möglich, dass die poröse Oberfläche wenigstens bereichsweise mit einer auf das Sintern nachfolgenden abtragenden chemischen oder physikalischen Behandlung weiter modifiziert ist.

Bevorzugt ist in diesem Zusammenhang beispielsweise eine Säurebehandlung, beispielsweise unter Zuhilfenahme von konzentrierter Schwefelsäure, und/oder Salzsäure und/oder einer anderen starken Säure bei erhöhter Temperatur (beispielsweise 100°C-300°C) und über einen Zeitraum von mehr als einer Minute.

Bevorzugt ist es in diesem Zusammenhang aber auch und besonders, dass diese nachfolgende Behandlung eine wenigstens bereichsweise erfolgende Salzschmelzenmodifikation umfasst, bevorzugtermassen indem das Implantat an der Oberfläche durch eine Salzschmelze durch Ätzung strukturiert wird, wobei insbesondere bevorzugt bei der Ätzung in der Salzschmelze im wesentlichen ausschliesslich eine Abtragung von Material erfolgt.

Insbesondere im Zusammenhang mit der Verwendung von anorganischem Strahlmittel, insbesondere wenn dies als feines Strahlmittel in einer Mischung verwendet wird, wird durch die nachträgliche Behandlung in der Salzschmelze sichergestellt, dass noch auf dem Implantat befindliches Strahlmittel in der Salzschmelze entfernt wird. Während mit anderen Worten das grobe Strahlmittel bereits beim Sintern entfernt wird, kann so auch noch das feine Strahlmittel, welches typischerweise nach den Strahlen des Grünlings noch auf der Oberfläche vorhanden ist, im wesentlichen restlos entfernt werden.

Bei der Salzschmelze kann es sich um eine Salzschmelze aus Alkali- und/oder Erdalkali-Nitraten, Hydroxiden oder Halogenen, oder einer Mischung dieser Salze handeln. Bevorzugt ist es, dass es sich bei der Salzschmelze, um eine Salzschmelze mit wenigstens einem Hydroxid, insbesondere mit wenigstens einem Alkali- und/oder Erdalkali-Hydroxid, handelt, oder dass es sich bei der Salzschmelze um eine Salzschmelze ausschliesslich bestehend aus einem oder mehreren Hydroxiden, insbesondere aus einem oder mehreren Alkali- und/oder Erdalkali-Hydroxiden handelt.

So kann es sich bei der Salzschmelze um eine Salzschmelze aus Kaliumhydroxid und/oder Natriumhydroxid und/oder Lithiumhydroxid handeln.

Es kann sich bei der Salzschmelze auch um eine Salzschmelze mit wenigstens einem Chlorid, insbesondere mit wenigstens einem Alkali- und/oder Erdalkali-Clorid, handeln, oder es kann sich bei der Salzschmelze um eine Salzschmelze ausschliesslich bestehend aus einem oder mehreren Chloriden, insbesondere aus einem oder mehreren Alkali- und/oder Erdalkali-Chloriden handeln.

Bevorzugtermassen handelt es sich bei der Salzschmelze zur Oberflächenmodifikation um eine binäre Salzschmelze aus Kaliumhydroxid und Natriumhydroxid oder aus Kaliumchlorid und Lithiumchlorid, dies insbesondere bevorzugt in einem Verhältnis von 2 : 1 - 0.5 : 1, vorzugsweise im Bereich von 1.5 : 1 - 0.75 : 1, insbesondere bevorzugt im Bereich von 1:1 oder 7:5, wobei bevorzugtermassen bei einer Temperatur im Bereich von 100 - 600 °C, insbesondere im Bereich von 150 - 250 °C gearbeitet wird.

Die Oberfläche kann bei dieser Behandlung in einer Salzschmelze wenigstens bereichsweise über eine Dauer von 10 Minuten bis 300 Stunden, bevorzugt von wenigstens 2 Stunden, vorzugsweise von 10 bis 100 Stunden, insbesondere von 25 bis 35 Stunden einer Salzschmelze ausgesetzt werden.

Bevorzugtermassen besteht das Implantat aus Keramik, es kann aber, wie bereits erläutert, auch auf metallischer Basis beruhen oder eine Kombination dieser beiden Materialien aufweisen.

Insbesondere bevorzugt handelt es sich um ein Implantat, welches Zirkonoxid enthält, welches gegebenenfalls zusätzlich mit Yttriumoxid und Hafniumoxid, versetzt ist, und/oder dass dieses Aluminiumoxid enthält, welches gegebenenfalls zusätzlich mit Siliziumdioxid, Eisen(III)oxid und/oder Natriumoxid versetzt ist, und/oder dass dieses Siliziumnitrid enthält, welches gegebenenfalls zusätzlich mit Siliziumdioxid, Eisen(III)oxid und/oder Natriumoxid versetzt ist, und/oder dass dieses Titanoxid enthält und/oder dass es aus Mischungen aus den genannten Materialien gebildet ist.

Zusätzlich ist es möglich, das Verfahren so durchzuführen, dass der Grünling während des kaltisostatischen Pressens, Giessens und/oder Spritzens durch Modifikation der Oberfläche des kaltisostatischen Press- Giess oder Spritzwerkzeuges vor dem Pressen, Giessen oder Spritzen des Ausgangsmaterials zum Grünling mit einem Strahlmittel an seiner Oberfläche verändert und/oder vorbereitet wird. Auch ist es möglich, dass der Grünling während des kaltisostatischen Pressens, Giessens und/oder Spritzens durch Zugabe eines Platzhaltermaterials zum Ausgangsmaterial wenigstens an seiner Oberfläche vor dem Sintern verändert und/oder vorbereitet wird. Die Modifikation der Werkzeugoberfläche mit einem Platzhalter kann durch die Behaftung der Werkzeugoberfläche mit dem Platzhalter erfolgen. Die temporäre Anbindung des Strahlmittels respektive Platzhaltermaterials an die Werkzeugoberfläche kann mit Bindern, beispielsweise organischen Bindern wie beispielsweise PVA oder auch mit Wachsen erfolgen.

In beiden Fällen erfolgt diese Behandlungen so dass die Struktur der Oberfläche des kaltisostatischen Press-, Giess-, oder Spritzwerkzeuges nach dem kaltisostatischen Pressen, Giessen oder Spritzen in der Oberfläche des Grünlings abgebildet ist, respektive dass das anschliessend entfernte Platzhaltermaterial die Oberfläche vorbereitet.

Bevorzugtermassen wird das Platzhaltermaterial selektiv nur im Oberflächenbereich angeordnet, insbesondere bevorzugt indem in einem ersten Schritt Ausgangsmaterial mit Platzhaltermaterial in die Form (bevorzugtermassen so, dass es in der Form im zukünftigen Oberflächenbereich des Implantates angeordnet ist) zugeführt wird und anschliessend in einem zweiten Schritt Ausgangsmaterial ohne Platzhaltermaterial. Im Gegensatz zu Verfahren, welche aber ohnehin nur aus anderen Bereichen, nicht aus dem Bereich der Herstellung von Implantaten bekannt sind (vergleiche beispielsweise wie DE 102 24 671 C1), bei welchen im gesamten Körper eine Porosität durch Platzhaltermaterial in der gesamten Masse vorgesehen wird, ist es also bevorzugt, das Implantat im Kern ohne derartige Platzhalter auszugestalten, da ansonsten in diesem Kern keine genügende Stabilität erreicht werden könnte.

Bevorzugtermassen handelt es sich beim Platzhaltermaterial um hochschmelzende organische oder anorganische Verbindungen, niedrigschmelzende Metalle, insbesondere bevorzugt um Carbamid (CH₄N₂O(H₂N-CO-NH₂)), Biuret (C₂H₅N₃O₂), Melamin (C₃H₆N₆), Melaminharz, Ammoniumkarbonat ((NH₄)CO₃H₂O) oder Ammoniumbikarbonat (NH₄HCO₃) oder Mischungen davon handelt. In Bezug auf mögliche Materialien als Platzhalter wird verwiesen auf den Offenbarungsgehalt der DE 102 24 671 C1, welche diesbezüglich in die vorliegende Offenbarung ausdrücklich eingeschlossen wird.

Weitere bevorzugte Ausführungsformen sind in den abhängigen Ansprüchen beschrieben.

Die Lösung dieser Aufgabe wird zusammenfassend dadurch erreicht, dass die strukturierte respektive poröse Oberfläche wenigstens bereichsweise während des CIM respektive MIM Prozesses am sogenannten Grünling, d.h. am Zwischenprodukt nach dem Giessen oder Spritzen und vor dem endgültigen Sintern oberflächenmodifiziert wird respektive das Resultat einer Oberflächenmodizierung ist. Die Aufgabe wird also durch eine spezifisch behandelte und dadurch spezifische Eigenschaften aufweisende Oberfläche des Implantates gelöst, wobei die Behandlung sowohl über die gesamte Implantatoberfläche als auch von partiellen Anteilen der Implantatoberfläche erfolgen kann.

Im Rahmen dieser Erfindung ist der Hauptsache von Implantaten die Rede, welche auf keramischen Materialien beruhen. Es ist aber gleichermassen möglich, Implantate auf metallischer Basis unter Zuhilfenahme der nachfolgend beschriebenen Prozesse zu strukturieren. Entsprechend ist es auch möglich, ein metallisches Implantat vorzusehen, welches eine strukturierte respektive poröse Oberfläche aufweist, welche wenigstens bereichsweise während des CIM respektive MIM Prozesses am sogenannten Grünling, d.h am Zwischenprodukt nach dem kaltisostatischen Pressen, Giessen oder Spritzen und vor dem endgültigen Sintern oberflächenmodifiziert wird respektive das Resultat einer Oberflächenmodizierung ist. Zusätzlich ist die Optimierung der Oberflächenstruktur durch thermische Wärmebehandlungen (Entbindern, Sintern, HIP) möglich. Sämtliche anfolgend beschriebenen Ausführungsformen liessen sich entsprechend gleichermassen auf metallische Materialien anwenden wie beispielsweise Implantate auf Basis von Titan, Zink, Niob, Tantal oder entsprechende Legierungen.

Der Kern der Erfindung besteht somit darin, dass überraschenderweise festgestellt wurde, dass insbesondere Grünlinge auf Basis von Keramik(schlicker) aber auch metall(schlicker)basierte Grünlinge unter Verwendung verschiedener Strahlmittel vor dem Sintern an der Oberfläche derart modifiziert werden können, dass sie anschliessend hervorragende Osteointegration resp. Osseointegration zeigen. Es zeigt sich, dass die Osteointegration resp. Osseointegration einer derart bearbeiteten Oberfläche besser ist, als die entsprechenden Werte für säuremodifizierte Oberflächen und/oder Oberflächen insbesondere von mechanisch hergestellten Keramiken, welche nur durch Sandstrahlen mit einer Makrorauhigkeit versehen wurden oder von Keramiken, die mittels CIM hergestellt wurden und nach dem endgültigen Sintern durch Sandstrahlen mit einer Makrorauhigkeit versehen wurden.

Der sogenannte Grünling wird also vor dem abschliessenden Sintern oder vorzugsweise vor einer Wärmebehandlung vor der Entbinderung an der Oberfläche durch Strahlen mit verschiedenen Strahlmitteln strukturiert. Dies kann beispielsweise durch einen definierten Strahlprozess erfolgen. Weiterhin besteht die Möglichkeit, das Giess- oder Spritzwerkzeug vor dem Giessen oder Spritzen mit dem Strahlmittel zu belegen oder zu behandeln. Geeignete Strahlmittel sind alle bekannten abrasiven oder oberflächenverdichtenden oder natürliche Strahlmittel, je nach gewünschter Rauheit bzw. Porosität der Oberfläche. Dabei wird auch gefunden, dass die erfindungsgemäss hergestellten Oberflächen teilweise eingetragene Bestandteile der verwendeten Strahlmittel enthalten können. Vorteilhafterweise können erfindungsgemäss weitere Strahlmittel verwendet werden, die vor dem abschliessenden Sintern rückstandslos entfernt werden können. Derartig geeignete Strahlmittel sind beispielsweise die genannten organischen Strahlmittel. Diese Strahlmittel können vor dem abschliessenden Sintern oder vorzugsweise vor einer Wärmebehandlung vor der Entbinderung rückstandsfrei bei Temperaturen bis max. 600°C oder max. 300°C entfernt werden. Vorteilhaft ist dabei, diese Behandlung in einer oxidierenden oder reduzierenden oder inerten Atmosphäre durchzuführen. Besonders vorteilhaft ist Ammoniumbikarbonat, welches schon bei 65°C von der Oberfläche des Grünlings sublimiert und die gewünschte Struktur in der Oberfläche hinterlässt. Die Grössenordung also die Korngrösse der Strahlmittel bestimmt die Grössenordung der Oberflächenstrukturierung. Typische Korngrössen sind in den Bereichen 10 - 200 µm, vorzugsweise 50 - 110 µm. Diese Strahlmittel wirken rein oberflächenstrukturierend, (die erfindungsgemäss hergestellte Oberfläche enthält dabei keine Rückstände der Strahlmittel). Die entstehende topologische Struktur entspricht dabei bei entsprechender Einstellung der Bedingungen und bei entsprechender Materialwahl des Implantats einer Makrorauhigkeit, das heisst vorzugsweise einer Rauhigkeit mit einer Grössenordnung 1 µm bis 50 µm, vorzugsweise 1 µm - 10 µm.

Diese makrostrukturierte Oberfläche kann zusätzlich mikrostrukturiert werden, beispielsweise mit einer Behandlung in einer Salzschmelze, wie sie z.B. in der CH 01339/06 beschrieben ist.

Zusätzliche Beschichtungen wie beispielsweise aus Apatit sind nicht erforderlich und bevorzugtermassen auch nicht vorhanden.

Bei der Keramik kann es sich um unterschiedliche Typen handeln, wobei diese aus dem Stand der Technik bekannt sind. Beispielsweise kann eine Keramik verwendet werden, welche Titanoxid oder Zirkonoxid enthält, welches gegebenenfalls zusätzlich mit Yttriumoxid und/oder Hafniumoxid, versetzt ist. Vergleiche dazu beispielsweise die US 6,165,925, deren Offenbarung hinsichtlich der Zusammensetzung und der Herstellung von solchen Keramiken auf Basis von Zirkonoxid ausdrücklich in den Offenbarungsgehalt der vorliegenden Beschreibung eingeschlossen werden soll.

Alternativ ist es möglich, Keramiken zu verwenden, welche Aluminiumoxid enthalten, welches gegebenenfalls zusätzlich mit Siliziumdioxid, Eisen(III)oxid und/oder Natriumoxidversetzt ist. Ebenfalls ist es möglich, eine Keramik zu verwenden, welche Siliziumnitrid enthält, welches gegebenenfalls zusätzlich mit Siliziumdioxid, Eisen(III)oxid und/oder Natriumoxid versetzt ist. Auch Keramiken basierend auf Mischungen oder Mehrschichtsystemen auf Basis der genannten Materialien sind möglich.

Gemäss einer bevorzugten Ausführungsform handelt es sich beim Implantat um ein Dentalimplantat, dessen im implantierten Zustand dem Knochen und/oder Weichgewebe ausgesetzte Oberfläche wenigstens bereichsweise mit Hilfe des beschriebenen Prozesses makrostrukturiert ist und die Makrostruktur noch mit einer Mikrostruktur unterlegt sein kann, die beispielsweise salzschmelzen- oder säurenmodifiziert ist.

### KURZE ERLÄUTERUNG DER FIGUREN

Die Erfindung soll nachfolgend anhand von Ausführungsbeispielen im Zusammenhang mit den Zeichnungen näher erläutert werden. Es zeigen:
- Fig. 1-3: Oberflächentopographie eines keramischen Implantates nach dem Spritzen und nach der Bestrahlung mit organischem Strahlmittel vor dem Sintern in verschiedenen Auflösungen;
- Fig. 4-6: Oberflächentopographie eines keramischen Implantates nach dem Spritzen und nach der Bestrahlung mit anorganischem Strahlmittel vor dem Sintern in verschiedenen Auflösungen;
- Fig. 7: Oberflächenaufnahmen von Beispiel 3 in unterschiedlichen Auflösungen vor dem Ätzen;
- Fig. 8: Oberflächenaufnahmen von Beispiel 3 in unterschiedlichen Auflösungen nach dem Ätzen;
- Fig. 9: Oberflächenaufnahmen von Beispiel 4 in unterschiedlichen Auflösungen vor dem Ätzen;
- Fig. 10: Oberflächenaufnahmen von Beispiel 4 in unterschiedlichen Auflösungen nach dem Ätzen; und
- Fig. 11: Oberflächenaufnahme eines Grünlings nach dem Strahlen.

### WEGE ZUR AUSFÜHRUNG DER ERFINDUNG

Die vorliegende Erfindung beschreibt die Möglichkeit, die Oberfläche von Implantaten, die insbesondere aus keramischen aber auch aus metallischen Materialien gefertigt werden, zu strukturieren. Zweck der Oberflächenstrukturierung sind eine bessere Verankerung der Implantate im Hartgewebe, eine verbesserter Verbund zwischen Hartgewebe und Implantatoberfläche, ein besserer Verbund zwischen Weichgewebe und Implantatoberfläche und eine verbesserte Interaktion der Implantatoberfläche an der Grenzfläche Implantatoberfläche Hartgewebe und/oder Weichgewebe.

Die Herstellung der Zirkonoxid- Titanoxid- und/oder Aluminiumoxid und/oder Mischkeramik für Implantate, auch unter Zuhilfenahme von CIM oder MIM, ist grundsätzlich im Stand der Technik bekannt und soll entsprechend nicht weiter ausgeführt werden. In diesem Zusammenhang sei beispielhaft auf die Offenbarung der oben genannten Dokumente Bezug genommen.

Vorzugsweise bezieht sich die Erfindung auf Implantate, welche im Hart- und/oder Weichgewebe verankert werden und dem temporären oder permanenten Ersatz oder Support von unfall-, abnutzungs-, mangelerscheinungs- oder krankheitsgeschädigten oder anderswie degenerierten Teilen des Bewegungsapparates unter Einschluss des Kauapparates, insbesondere des Dentalbereiches mit den zugehörigen auch ästhetischen Aspekten, dienen. So sind beispielsweise Hüft- und Kniegelenksprothesen, Wirbelsäulenimplantate und Dentalimplantate seit vielen Jahren im klinischen Einsatz. Die Aufgabe der verbesserten Osteointegrationseigenschaften respektive Osseointegrationseigenschaften wird erfindungsgemäss durch eine entsprechende Oberflächenstruktur respektive Oberflächenbehandlung der (Keramik-)Oberfläche des Implantates gelöst, wobei die Behandlung sowohl über die gesamte Implantatoberfläche als auch von partiellen Bereichen der Implantatoberfläche erfolgen kann. Durch eine derartige Oberflächenstrukturierung wird gewährleistet, dass die ansonsten bioinerten Keramiken, wie vorzugsweise Zirkonoxid, Titanoxid oder Aluminiumoxid oder Mischungen davon, in das Hart- und /oder Weichgewebe integriert werden können.

Die strukturelle und funktionelle Verankerung, z. B. eines Zahnimplantats, im Knochen wird in der Regel durch Anbringen einer Makrorauhigkeit, und/oder einer, gegebenenfalls zusätzlichen, Mikrorauhigkeit, erreicht. Die Makrorauhigkeit kann beispielsweise nach dem Stand der Technik mit einem mechanischen Strahlprozess, die Mikrorauhigkeit nachfolgend beispielsweise entweder in einem additiven Prozess mittels Plasmatechnik, oder in einem subtraktiven Prozess durch chemische oder Salzschmelzen-Ätzung auf der Oberfläche bewirkt werden. Wie fest das Implantat im Knochen verankert ist, kann mit mechanischen Messungen festgestellt werden. Zahlreiche Untersuchungen haben gezeigt, dass die genügende Verankerung eines Implantates im Knochen in hohem Mass von der Oberflächenbeschaffenheit des Implantats, insbesondere von der Rauhigkeit an dessen Oberfläche, abhängt.

Die vorliegende Erfindung beschreibt eine spezifische und neuartig erzeugte Rauhigkeit für eine bevorzugtermassen vergrösserte wirksame Oberfläche zur besseren Osteointegration von Implantaten, welche aus Keramiken, vorzugsweise aus Titanoxid, Zirkonoxid oder Aluminiumoxid oder Mischungen davon hergestellt sind. Diese erfindungsgemäss biologisch wirksame Oberfläche kann durch Strahlen des Grünlings nach dem Giessen oder Spritzen und vor dem endgültigen Sintern während des CIM respektive MIM Prozesses mit zusätzlicher mechanischer anschliessender oder vorgängiger chemischer Behandlung, beispielsweise Ätzung o.ä. oder durch eine Kombination solcher Verfahren hergestellt werden.

Die erfindungsgemässe Oberfläche kann hergestellt werden, indem man den Grünling vor dem abschliessenden Sintern an der Oberfläche durch Strahlen mit verschiedenen Strahlmitteln behandelt bis eine entsprechende Oberflächenstrukturierung entstanden ist. Dies kann beispielsweise durch einen definierten Strahlprozess erfolgen.

Weiterhin besteht die Möglichkeit, das isostatische Press- oder Giess- oder Spritzwerkzeug vor dem Giessen oder Spritzen mit dem Strahlmittel zu belegen oder zu behandeln.

Wie erwähnt sind alle bekannten abrasiven oder oberflächenverdichtenden Strahlmittel geeignet, wie metallische Strahlmittel, keramische Strahlmittel oder natürliche Strahlmittel in verschiedenen Korngrössen, je nach gewünschter Rauheit bzw. Porosität der Oberfläche. Dabei wird auch gefunden, dass die erfindungsgemäss hergestellten Oberflächen teilweise eingetragenen Bestandteile der verwendeten Strahlmittel enthalten können. Vorteilhafterweise können weitere Strahlmittel verwendet werden, die vor dem abschliessenden Sintern rückstandslos entfernt werden können. Derartig geeignete Strahlmittel sind beispielsweise Eis(kugeln oder -partikel), organische Strahlmittel oder speziell Carbamid, Melaminharz, Biuret, Melamin, Ammoniumkarbonat und Ammoniumbikarbonat. Diese Strahlmittel werden vor dem abschliessenden Sintern oder vorzugsweise vor einer Wärmebehandlung vor der Entbinderung rückstandsfrei bei Temperaturen bis max. 600°C entfernt. Vorteilhaft ist dabei, diese Behandlung in einer oxidierenden oder reduzierenden oder inerten Atmosphäre durchzuführen. Die Grössenordnung der/des Strahlmittels bestimmt die Grössenordung der Oberflächenstrukturierung. Bei Mischung aus zwei verschiedenen Mitteln mit verschiedenen Grössen entstehen somit "bimodale" Strukturierungen mit zwei verschiedenen Struktur-Dimensions-Anteilen, einer Feinstruktur und einer Grobstruktur.

### Serie 1:

### Beispiel 1

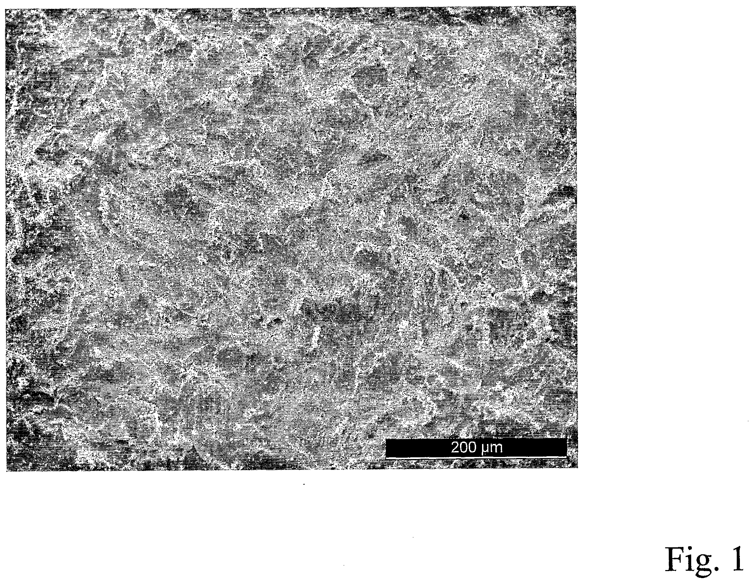

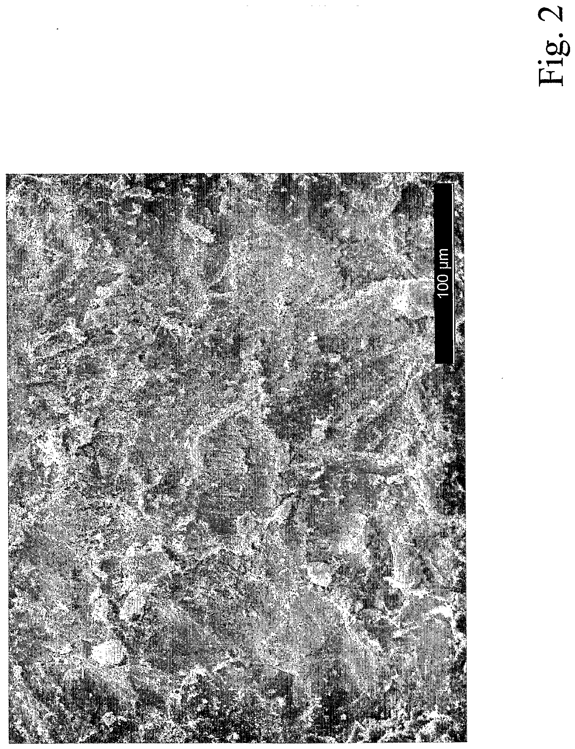

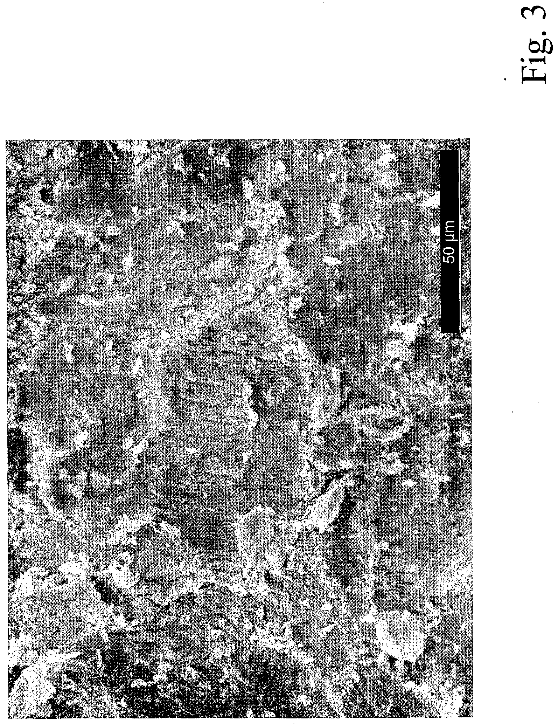

Ein Grünling in Form eines zylindrischen Zahnimplantates mit 10 mm Länge und 4 mm Durchmesser wurde aus yttriumstabilsiertem Zirkonoxidpulver gespritzt. Nach dem Spritzen und vor dem Sintern wurde die Oberfläche mit einem Gemisch aus Pfirsich- und Aprikosenkernen mit einer Korngrösse von 100 - 150 µm mit einem Druck von 0.8 bar 50 s gestrahlt. Die dabei entstehende Oberfläche wurde mit Rasterelektronenmikroskopie untersucht. Die durch das Strahlen erzeugte Oberflächentopografie ist in den Figuren 1, 2 und 3 in unterschiedlichen Auflösungen dargestellt. Die dabei erzeugte Makrorauhigkeit führt nach dem Sintern zu einer guten Osseointegration des Implantates.

### Beispiel 2

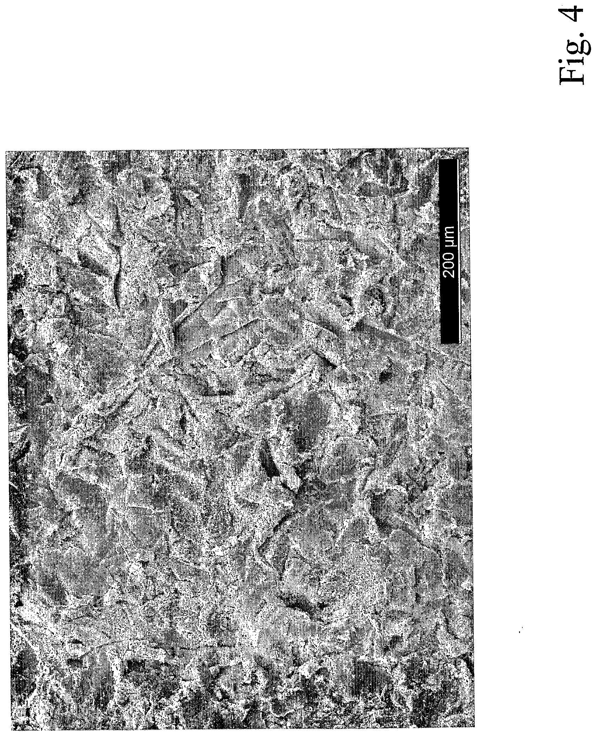

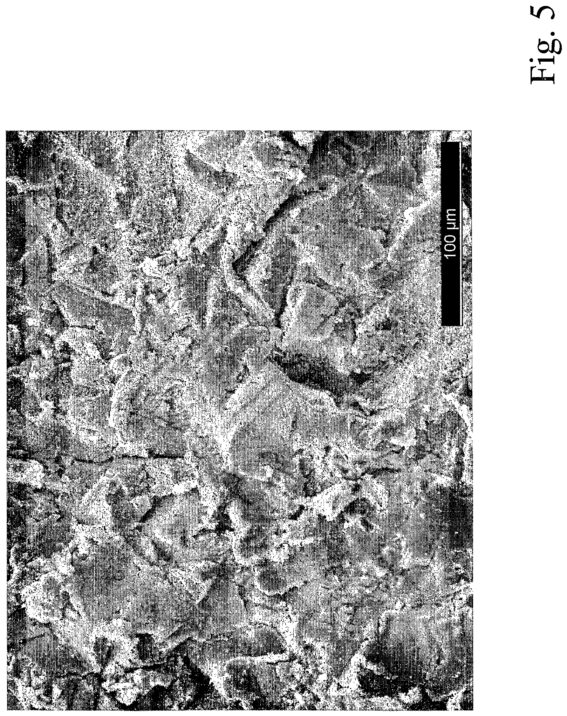

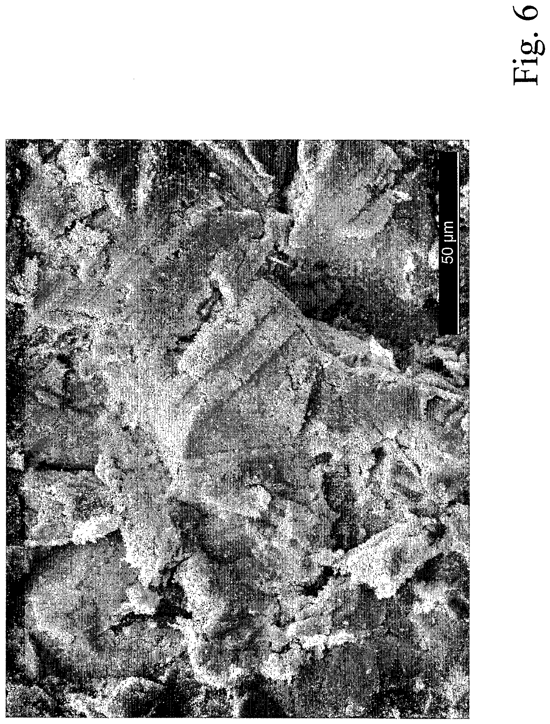

Ein Grünling in Form eines zylindrischen Zahnimplantates mit 10 mm Länge und 4 mm Durchmesser wurde aus yttriumstabilsiertem Zirkonoxidpulver gespritzt. Nach dem Spritzen und vor dem Sintern wurde die Oberfläche mit Aluminiumoxid mit einer Korngrösse von etwa 250 µm mit einem Druck von 0.8 bar 50 s gestrahlt. Die dabei entstehende Oberfläche wurde mit Rasterelektronenmikroskopie untersucht. Die durch das Strahlen erzeugte Oberflächentopografie ist in den Figuren 4, 5 und 6 in unterschiedlichen Auflösungen dargestellt. Die dabei erzeugte Makrorauhigkeit führt nach dem Sintern zu einer guten Osseointegration des Implantates.

### Serie 2:

In einer zweiten Serie von Experimenten wurden die Grünlinge unter Verwendung eines Strahlmittels, welches zwei verschiedene Materialien mit unterschiedlicher Korngrösse enthielt, vor dem Sintern bearbeitet. Generell werden dabei folgende Verfahrensführung und Einstellungen der Parameter bevorzugt:

### Bevorzugte Parameter:

1. Durchgang mit 2/3 Vol. organischem Mittel (Pfirsichkerne und/oder Aprikosenkerne in entsprechend gemahlener Form) 0.3 bis 0.6 mm Körngrosse und 1/3 Vol. Al₂O₃ - 220 mesh (circa 0.07 mm Partikelgrösse). Die beiden Komponenten liegen als Gemisch vor und werden gleichzeitig gestrahlt.

| | | |
|---|---|---|
| Druck: | 3 bar bis | 5 bar |
| Expositions- Strahlungs-Zeit: | 25 sec bis | 45 sec |
| Abstand, Düse zum Implantat: | 25 mm bis | 80 mm |
| Bohrungsdurchmesser der Düse: | 1.2 mm bis | 2.0 mm |

2. Durchgang mit Al₂O₃ mesh 220, damit können gröbere Rückstande des organischen

**Mittels entfernt werden.**

| | | |
|---|---|---|
| Druck: | 0.2 bar bis | 0.4 bar |
| Expositions- Strahlungs-Zeit: | 15 sec bis | 25 sec |
| Abstand, Düse zum Implantat: | 30 mm bis | 50 mm |
| Bohrungsdurchmesser der Düse: | 0.8 mm bis | 1.0 mm |

**Besonders bevorzugt für den 2.Durchgang sind:**

| | |
|---|---|
| Druck: | 0.2 bar |
| Expositions- Strahlungs-Zeit: | 20 sec |
| Abstand, Düse zum Implantat: | 30 mm |
| Bohrungsdurchmesser der Düse: | 1.0 mm |

Generell können die in der Folge dargestellten Parameter gewählt werden:
1. Durchgang mit 2/3 Vol. organischem Mittel (Pfirsichkerne und/oder Aprikosenkerne in entsprechend gemahlener Form) 0.3 bis 0.6 mm Körngrosse und 1/3 Vol. Al₂O₃ - 220 mesh.

| | | |
|---|---|---|
| Druck: | 2 bar bis | 7 bar |
| Expositions- Strahlungs-Zeit: | 15 sec bis | 65 sec |
| Abstand, Düse zum Implantat: | 25 mm bis | 80 mm |
| Bohrungsdurchmesser der Düse: | 1.2 mm bis | 2.0 mm |

2. Durchgang mit Al₂O₃ mesh 220.

| | | |
|---|---|---|
| Druck: | 0.2 bar bis | 0.8 bar |
| Expositions- Strahlungs-Zeit: | 10 sec bis | 35 sec |
| Abstand, Düse zum Implantat: | 30mm bis | 50mm |
| Bohrungsdurchmesser der Düse: | 0.8mm bis | 1.2mm |

### Beispiel 3

Ein Grünling in Form eines zylindrischen Zahnimplantates mit 10 mm Länge und 4 mm Durchmesser wurde aus yttriumstabilisiertem Zirkonoxidpulver gespritzt. Nach dem Spritzen und vor dem Sintern wurde die Oberfläche mit einem Gemisch aus Pfirsich- und Aprikosenkernen 2/3 Vol. (organisches Mittel) 0.3 bis 0.6 mm Körngrosse und 1/3 Vol. Al₂O₃ - 220 mesh mit einem Druck von 3.0 bar 45 s gestrahlt.

Anschliessend 2. Strahl-Durchgang mit Al₂O₃ mesh 220, damit können gröbere Rückstande des organisches Mittels entfernt werden, mit einem Druck von 0.8 bar 50 s.

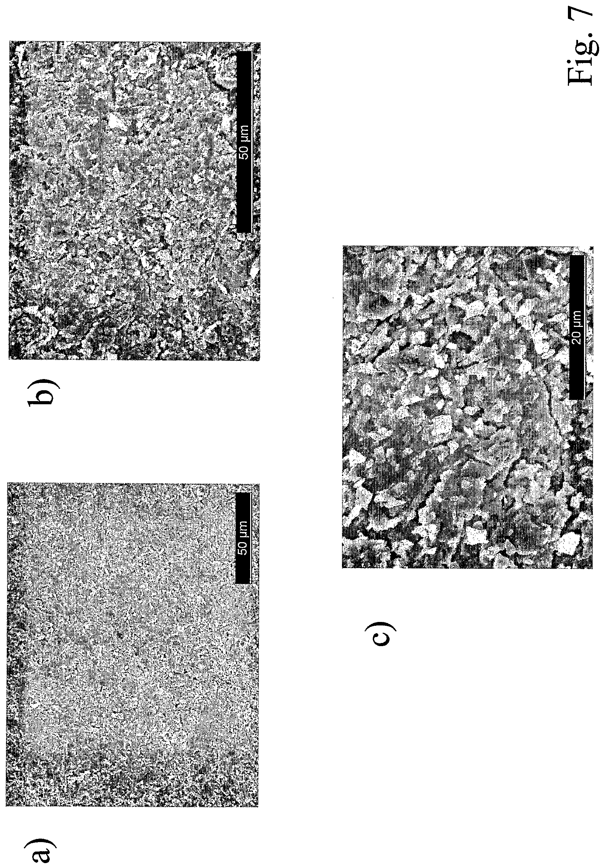

Die dabei entstehende Oberfläche wurde mit Rasterelektronenmikroskopie untersucht. Die durch das Strahlen erzeugte Oberflächentopografie ist in der Fig. 7 (a-c) dargestellt. Die dabei erzeugte Makrorauhigkeit führt nach dem Sintern zu einer guten Osseointegration des Implantates.

Die Werte der Rauheitsmessungen der Oberfläche des so hergestellten Implantates im Zustand vor dem Ätzen, am Gewindegrund gemessen, ergeben folgende Werte:

**Messwerte in µm**

| Sa | Sq | St | Sk | Rt | Rq | Ra |
|---|---|---|---|---|---|---|
| 1.05 | 1.25 | 6.42 | 3.38 | 8.67 | 1.41 | 1.10 |

Messparameter (auch in allen weiteren Messungen verwendet): Gaussfilter mit cut off =110µm; Messfeld ca. 770 µm x 770 µm. Objektiv L20X, Stitchen 1x1; Konfokalmikroskop 3 dimensionale Messmethode, Gerät: white light microscopy µ-surf.

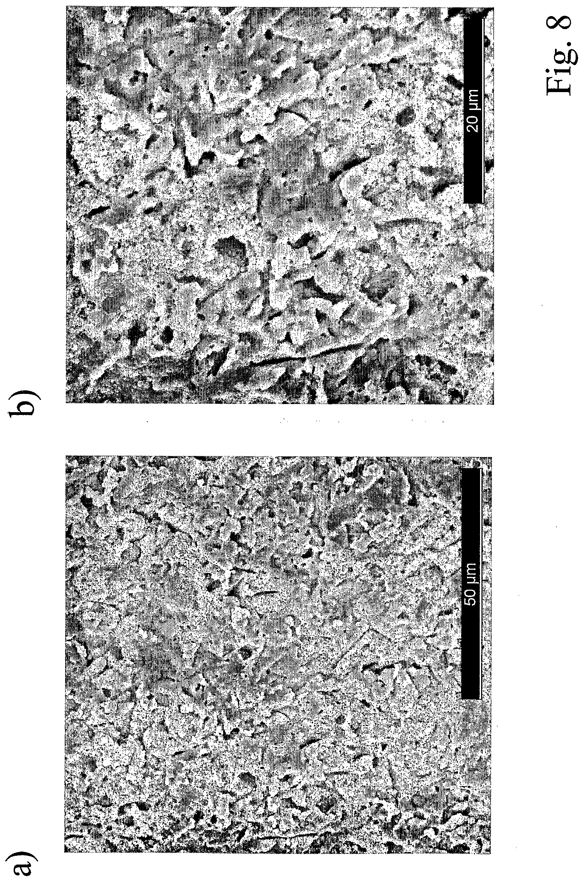

Anschliessend wurde das so hergestellte Implantat in einer Salzschmelze, bestehend aus 50% KOH und 50% LiOH (Gewichtsprozente) bei 200°C während 30 Stunden, geätzt. Die Oberflächenstruktur wurde dabei wesentlich verändert, wie sich dies aus Figur 8 (ab) erschliesst.

Die Werte der Rauheitsmessungen der Oberfläche des Implantates im nach dem Ätzen, am Gewindegrund gemessen, ergeben folgende Werte:

**Messwerte in µm**

| Sa | Sq | St | Sk | Rt | Rq | Ra | |
|---|---|---|---|---|---|---|---|
| 1.12 | 1.41 | 7.57 | 7.87 | 16.81 | 3.91 | 1.23 | nach dem Ätzen |

### Beispiel 4

Ein Grünling in Form eines zylindrischen Zahnimplantates mit 10 mm Länge und 4 mm Durchmesser wurde aus yttriumstabilsiertem Zirkonoxidpulver gespritzt. Nach dem Spritzen und vor dem Sintern wurde die Oberfläche mit einem Gemisch aus Pfirsich- und Aprikosenkernen 2/3 Vol. (organisches Mittel) 0.3 bis 0.6 mm Körngrosse und 1/3 Vol. Al₂O₃ - 220 mesh mit einem Druck von 3.0 bar 25 s gestrahlt.

Anschliessend 2. Strahl-Durchgang mit Al₂O₃ mesh 220, damit können gröberen Rückstande des organisches Mittels entfernt werden, mit einem Druck von 0.2 bar 20 s.

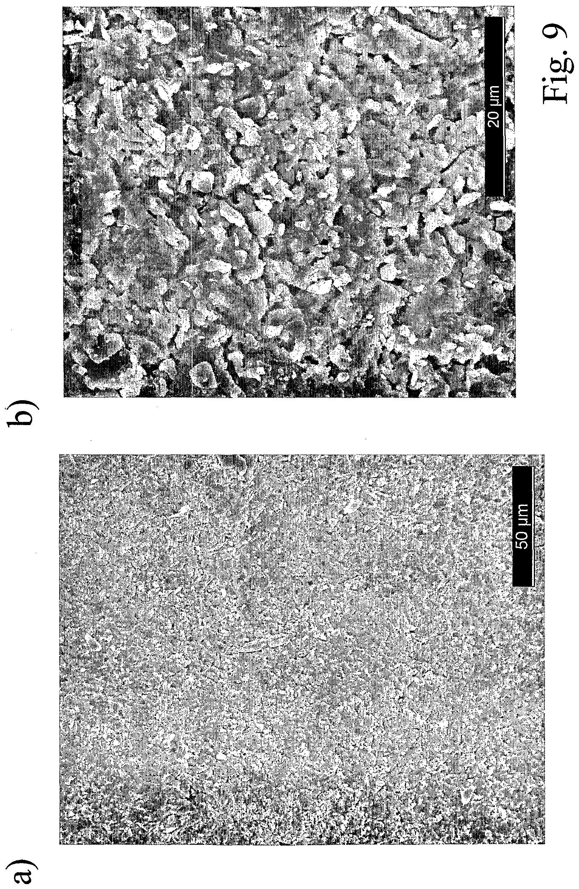

Die dabei entstehende Oberfläche wurde mit Rasterelektronenmikroskopie untersucht. Die durch das Strahlen erzeugte Oberflächentopografie ist in der Fig. 9 (a-b) dargestellt. Die dabei erzeugte Makrorauhigkeit führt nach dem Sintern zu einer guten Osseointegration des Implantates.

Die Werte der Rauheitsmessungen der Oberfläche des Implantates im vor dem Ätzen, am Gewindegrund gemessen, ergeben folgende Werte:

**Messwerte in µm**

| Sa | Sq | St | Sk | Rt | Rq | Ra | |
|---|---|---|---|---|---|---|---|
| 1.07 | 1.31 | 6.42 | 3.53 | 6.93 | 1.43 | 1.03 | Vor dem Ätzen |

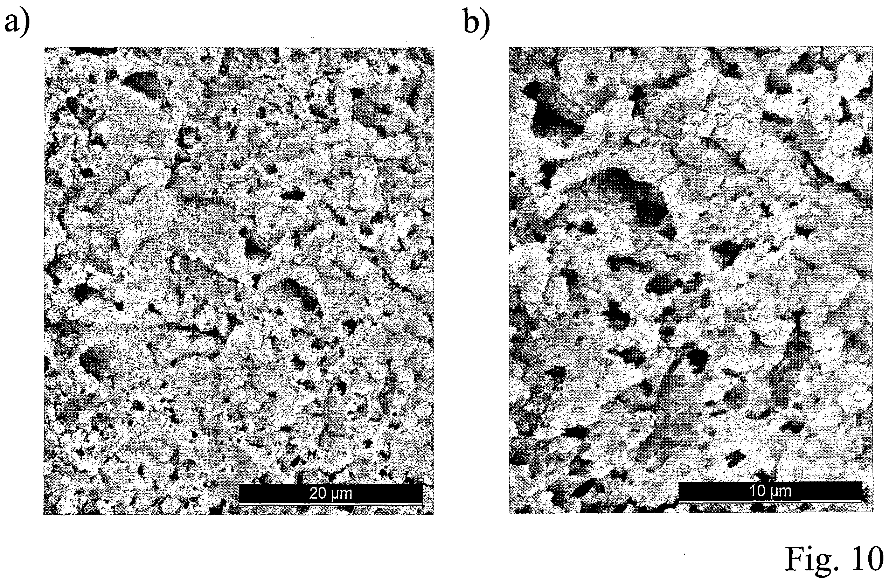

Anschliessend wurde das so hergestellte Implantat in einer Salzschmelze, bestehend aus 50% KOH und 50% LiOH (Gewichtsprozente) bei 200°C während 30 Stunden, geätzt. Die Oberflächenstruktur wurde dabei wesentlich verändert, wie sich dies aus Figur 10 (a-b) erschliesst.

Die Werte der Rauheitsmessungen der Oberfläche des Implantates im nach dem Ätzen, am Gewindegrund gemessen, ergeben folgende Werte:

**Messwerte in µm**

| Sa | Sq | St | Sk | Rt | Rq | Ra | |
|---|---|---|---|---|---|---|---|
| 1.95 | 1.53 | 17.39 | 4.17 | 41.11 | 4.58 | 1.80 | Nach dem Ätzen. |

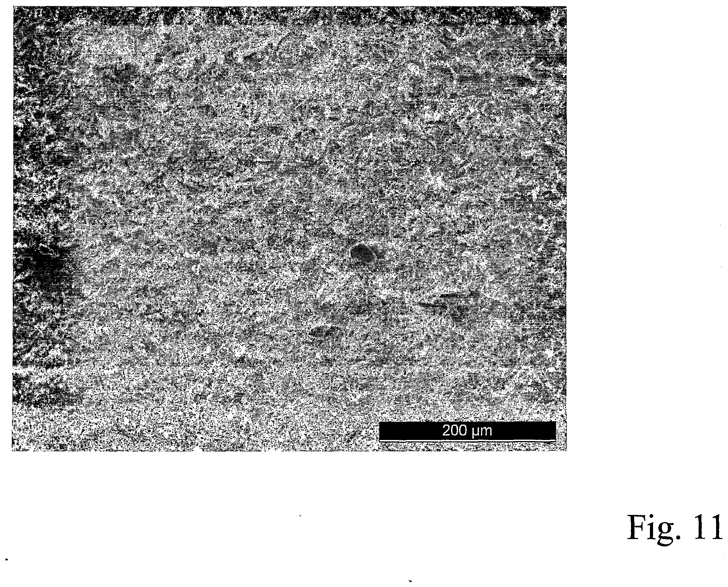

In Figur 11 ist ein Grünling vor dem Sintern dargestellt, dabei ist erkennbar, wie die organischen und anorganischen Rückstände des Strahlmittels auf der Oberfläche noch vorhanden sind.

## Patentansprüche

1. Verfahren zur Herstellung eines metallischen und/oder keramischen Implantates mit einer strukturierten, insbesondere porösen Oberfläche zum wenigstens teilweisen Einsetzen in Hartgewebe wie in einen Knochen und/oder in Weichgewebe, wobei das Implantat wenigstens bereichsweise unter Zuhilfenahme eines kaltisostatischen Pressens, Giessens und/oder Spritzens (CIM, MIM) zu einem Grünling mit anschliessendem Sintern zum Implantat hergestellt wird,
wobei
die Oberfläche vor dem Sintern derart verändert und/oder vorbereitet wird, dass nach dem Sintern eine makroporöse und/oder makrostrukturierte Oberfläche vorliegt,
wobei der Grünling nach dem kaltisostatischen Pressen, Giessen und/oder Spritzen und vor dem endgültigen Sintern durch Strahlen der Oberfläche des Grünlings modifiziert wird,
und wobei es sich beim Strahlmittel für das Strahlen um ein abrasives und/oder oberflächenverdichtendes Strahlmittel handelt,
**dadurch gekennzeichnet, dass**
als Strahlmittel eine Mischung aus zwei unterschiedlichen Strahlmitteln mit unterschiedlicher Korngrösse eingesetzt wird, wobei der Unterschied in der mittleren Korngrösse der unterschiedlichen Strahlmittel im Bereich von einem Faktor 5-10 liegt.

2. Verfahren nach Anspruch 1, **dadurch gekennzeichnet, dass** der Grünling nach dem Sintern ohne zusätzliche Nachbearbeitung eine makroporöse und/oder makrostrukturierte Oberfläche aufweist.

3. Verfahren nach Anspruch 1 oder 2, **dadurch gekennzeichnet, dass** es sich beim abrasiven und/oder oberflächenverdichtenden Strahlmittel um ein metallisches Strahlmittel wie Stahlkugeln, ein keramisches Strahlmittel wie Al₂O₃, ZrO₂, SiO₂, Ca Phosphate, TiO₂, NaO₂, CaO, MgO, ein organisches oder natürliches Strahlmittel wie Nussschalen oder Reis in verschiedenen Korn- und Splittergrössen, oder Mischungen der genannten Strahlmittel handelt, und vorzugsweise die Korngrösse des Strahlmittels in den Bereichen 0.01 - 0.25 mm und/oder 0.01 - 0.2 mm liegt
und/oder wobei es sich beim Strahlmittel für das Strahlen um organische Strahlmittel wie Stearate, Wachse, Paraffine oder bevorzugt Carbamid, Melaminharz, Biuret, Melamin, Ammoniumkarbonat und Ammoniumbikarbonat oder Mischungen davon handelt.

4. Verfahren nach Anspruch 3, **dadurch gekennzeichnet, dass** die Strahlmittel vor dem abschliessenden Sintern rückstandsfrei bei Temperaturen bis max. 600°C entfernt werden können, wobei diese Entfernung bevorzugtermassen in einer oxidierenden oder reduzierenden oder inerten Atmosphäre durchgeführt wird, wie insbesondere bevorzugt in unter O₂, N₂, NH₄, Ar, Mischungen davon oder im Vakuum, wobei vorzugsweise Ammoniumbikarbonat, schon bei 65°C von der Oberfläche des Grünlings sublimiert und die gewünschte Struktur in der Oberfläche hinterlässt.

5. Verfahren nach einem der Ansprüche 3-4, **dadurch gekennzeichnet, dass** der Strahldruck im Bereich von 0.2 - 7 bar, bevorzugtermassen zwischen 0.2-5 bar, insbesondere bevorzugt im Bereich von 0.8 bar liegt, und/oder dass die Strahlbehandlung während einer Zeitdauer zwischen 15 und 65 Sekunden, die bevorzugt zwischen 35 und 55 Sekunden, insbesondere bevorzugt im Bereich von 50 Sekunden durchgeführt wird, wobei bevorzugtermassen der Abstand von der Düse zum Implantat im Bereich von 25-80 mm, besondere zwischen 25 und 60 mm, insbesondere bevorzugt im Bereich von 30 mm gewählt wird, bei einem Bohrungsdurchmesser der Düse im Bereich von 0.8 -1.2mm, bevorzugtermassen im Bereich von 0.8-1.0mm.

6. Verfahren nach einem der Ansprüche 1 - 5, **dadurch gekennzeichnet, dass** ein erstes Strahlmittel in der Mischung vorhanden ist, welches eine mittlere Korngrösse im Bereich von 0.1-0.2 mm, bevorzugt im Bereich von 0.2-0.8 mm aufweist, wobei es sich dabei bevorzugt um ein organisches Strahlmittel, insbesondere aus Obstkernen, handelt, und dass ein zweites Strahlmittel in der Mischung vorhanden ist, welches eine mittlere Korngrösse im Bereich von 0.01-0.1 mm, bevorzugt im Bereich von 0.03-0.9 mm aufweist, wobei es sich dabei bevorzugt um ein anorganisches Strahlmittel, insbesondere auf Basis von Aluminiumoxid (Al2O3), handelt, wobei bevorzugtermassen das Verhältnis von erstem zu zweitem Strahlmittel im Bereich von 5:1 - 1: 5, bevorzugt im Bereich von 3:1 - 1:1 liegt, wobei vorzugsweise in zwei Schritten gestrahlt wird, wobei in einem ersten Schritt die genannte Mischung verwendet wird und in einem zweiten Schritt nur noch das Strahlmittel mit der geringeren Korngrösse, bevorzugt als ein anorganisches Strahlmittel, eingesetzt wird, wobei bevorzugtermassen der zweite Schritt unter einem wenigstens 5-10 mal kleineren Strahldruck gefahren wird.

7. Verfahren nach einem der Ansprüche 1-6, **dadurch gekennzeichnet, dass** beim Schritt unter Verwendung einer Mischung ein Druck im Bereich von 2-7 bar, bevorzugt 3-5 bar verwendet wird, und/oder die Zeit der Behandlung im Bereich von 15-65 Sekunden, bevorzugt im Bereich von 25-45 Sekunden liegt und/oder der Abstand von der Düse zum Implantat im Bereich von 25-80 mm liegt, und/oder der Bohrungsdurchmesser der Düse im Bereich von 1.2-2.0 mm liegt, und das bei einem gegebenenfalls vorhandenen zweiten Schritt ein Druck im Bereich von 0.2-0.8 bar, bevorzugt im Bereich von 0.2-0.4 bar liegt, und/oder die Zeit der Behandlung im Bereich von 10-35 Sekunden, bevorzugt im Bereich von 15-25 Sekunden liegt, und/oder der Abstand von der Düse zum Implantat im Bereich von 30-50 mm liegt, und/oder der Bohrungsdurchmesser der Düse im Bereich von 0.8 mm-1.2 mm liegt.

8. Verfahren nach einem der vorhergehenden Ansprüche, **dadurch gekennzeichnet, dass** die poröse Oberfläche wenigstens bereichsweise mit einer auf das Sintern nachfolgenden abtragenden chemischen oder physikalischen Behandlung weiter modifiziert ist.

9. Verfahren nach Anspruch 14, **dadurch gekennzeichnet, dass** die nachfolgende Behandlung eine wenigstens bereichsweise erfolgende Salzschmelzenmodifikation umfasst, indem das Implantat an der Oberfläche durch eine Salzschmelze durch Ätzung strukturiert wird, wobei insbesondere bei der Ätzung in der Salzschmelze im wesentlichen ausschliesslich eine Abtragung von Material erfolgt.

10. Verfahren nach Anspruch 15, **dadurch gekennzeichnet, dass** es sich bei der Salzschmelze um eine Salzschmelze aus Alkali- und/oder Erdalkali-Nitraten, Hydroxiden oder Halogenen, oder einer Mischung dieser Salze handelt.

11. Verfahren nach einem der vorhergehenden Ansprüche 15 oder 16, **dadurch gekennzeichnet, dass** es sich bei der Salzschmelze um eine Salzschmelze mit wenigstens einem Hydroxid, insbesondere mit wenigstens einem Alkali- und/oder Erdalkali-Hydroxid, handelt, oder dass es sich bei der Salzschmelze um eine Salzschmelze ausschliesslich bestehend aus einem oder mehreren Hydroxiden, insbesondere aus einem oder mehreren Alkali- und/oder Erdalkali-Hydroxiden handelt und/oder, dass es sich bei der Salzschmelze um eine Salzschmelze aus Kaliumhydroxid und/oder Natriumhydroxid und/oder Lithiumhydroxid handelt.

12. Verfahren nach einem der vorhergehenden Ansprüche 15 oder 16, **dadurch gekennzeichnet, dass** es sich bei der Salzschmelze um eine Salzschmelze mit wenigstens einem Chlorid, insbesondere mit wenigstens einem Alkali- und/oder Erdalkali-Clorid, handelt, oder dass es sich bei der Salzschmelze um eine Salzschmelze ausschliesslich bestehend aus einem oder mehreren Chloriden, insbesondere aus einem oder mehreren Alkali- und/oder Erdalkali-Chloriden handelt, wobei es sich vorzugsweise um eine binäre Salzschmelze aus Kaliumhydroxid und Natriumhydroxid oder aus Kaliumchlorid und Lithiumchlorid handelt, in einem Verhältnis von 2 : 1 - 0.5 : 1, vorzugsweise im Bereich von 1.5 : 1 - 0.75 : 1, insbesondere bevorzugt im Bereich von 1:1 oder 7:5, wobei bevorzugtermassen bei einer Temperatur im Bereich von 100 - 600 °C, insbesondere im Bereich von 150 - 250 °C gearbeitet wird, und/oder wobei die Oberfläche wenigstens bereichsweise über eine Dauer von 10 Minuten bis 300 Stunden, bevorzugt von wenigstens 2 Stunden, vorzugsweise von 10 bis 100 Stunden, insbesondere von 25 bis 35 Stunden einer Salzschmelze ausgesetzt wird.

13. Verfahren nach einem der vorhergehenden Ansprüche, **dadurch gekennzeichnet, dass** das Implantat aus Keramik besteht, wobei dieses Implantat vorzugsweise Zirkonoxid enthält, welches gegebenenfalls zusätzlich mit Yttriumoxid und Hafniumoxid, versetzt ist, und/oder dass dieses Aluminiumoxid enthält, welches gegebenenfalls zusätzlich mit Siliziumdioxid, Eisen(III)oxid und/oder Natriumoxid versetzt ist, und/oder dass dieses Siliziumnitrid enthält, welches gegebenenfalls zusätzlich mit Siliziumdioxid, Eisen(III)oxid und/oder Natriumoxid versetzt ist, und/oder dass dieses Titanoxid enthält und/oder dass es aus Mischungen aus den genannten Materialien gebildet ist.

## Claims

1. Method for the production of a metallic and/or ceramic implant with a structured, especially porous surface for at least partial insertion into hard tissue such as into a bone and/or into soft tissue, wherein the implant is produced at least area-wise by the aid of a cold-isostatic compression, casting and/or injection molding (CIM, MIM) to a green body with subsequent sintering to an implant, wherein
prior to sintering the surface is changed and/or prepared such that after the sintering a macroporous and/or macro-structured surface is present wherein the green body is modified after the cold-isostatic compression, casting and/or injection molding and prior to the final sintering by blasting of the surface of the green body,
wherein the blasting agent for the blasting is an abrasive and/or surface-densifying blasting agent
**characterized in that**
a mixture of two different blasting agents with a different particle size is used as a blasting agent, wherein the difference in the average particle size of the different blasting agents lies in the range of a factor 5-10.

2. Method according to claim 1, **characterized in that** after sintering, the green body has a macroporous and/or macro-structured surface without any additional posttreatment.

3. Method according to claim 1 or 2, **characterized in that** the abrasive and/or surface-densifying blasting agent is a metallic blasting agent such as steel balls, a ceramic blasting agent such as Al₂O₃, ZrO₂, SiO₂, Ca-phosphate, TiO₂, NaO₂, CaO, MgO, an organic or natural blasting agent such as nut shells or rice in various particle- and splitter sizes, or mixtures of said blasting agents, and wherein the particle size of the blasting agent lies in the ranges of 0.01-0.25 mm and/or 0.01-0.2 mm
and/or **characterized in that** the blasting agent for the blasting are ice balls, ice particles, organic blasting agents such as stearates, waxes, paraffines, or preferably carbamide, melamine resin, biuret, melamine, ammonium carbonate and ammonium bicarbonate or mixtures thereof.

4. Method according to claim 3, **characterized in that** the blasting agents can be removed without residues at temperatures up to max. 600°C prior to the final sintering, wherein this removal preferably is carried out in an oxidizing or reducing or inert atmosphere, such as especially preferably in under O₂, N₂, NH₄, Ar, mixtures thereof, or in vacuum, wherein preferably the ammonium bicarbonate already sublimes from the surface of the green body at 65°C and leaves behind the desired structure in the surface.

5. Method according to one of claims 3-4, **characterized in that** the blasting pressure lies in the range of 0.2-7 bar, preferably between 0.2-5 bar, especially preferably in the range of 0.8 bar, and/or that the blasting treatment is carried out during a time period between 15 and 65 seconds, preferably between 35 and 55 seconds, especially preferably in the range of 50 seconds, wherein preferably the distance from the jet to the implant is selected in the range of 25-80 mm, especially between 25 and 60 mm, especially preferably in the range of 30 mm, at a bore diameter of the jet in the range of 0.8-1.2 mm, preferably in the range of 0.8-1.0 mm.

6. Method according to one of claims 1-5, **characterized in that** a first blasting agent is present in the mixture, which has an average particle size in the range of 0.1-0.2 mm, preferably in the range of 0.2-0.8 mm, wherein it preferably is an organic blasting agent, especially of fruit kernels, and that a second blasting agent is present in the mixture, which has an average particle size in the range of 0.01-0.1 mm, preferably in the range of 0.03-0.9 mm, wherein it preferably is an anorganic blasting agent, especially on the basis of aluminium oxide (Al2O3), wherein preferably the ratio of the first to the second blasting agent is in the range of 5:1 - 1:5, preferably in the range of 3:1 - 1:1, wherein preferably the blasting is carried out in two steps, wherein in a first step said mixture is used and in a second step only the blasting agent with the smaller particle size is used, preferably as an anorganic blasting agent, wherein preferably the second step is carried out under an at least 5-10 times lower blasting pressure.

7. Method according to one of claims 10-12, **characterized in that** in the step of using a mixture a pressure within the range of 2-7 bar, preferably 3-5 bar is used, and/or the time of treatment is in the range of 15-65 seconds, preferably in the range of 25-45 seconds and/or the distance from the jet to the implant is in the range of 25-80 mm, and/or the bore diameter of the jet is in the range of 1.2-2.0 mm, and that in a possibly present second step a pressure lies in the range of 0.2-0.8 bar, preferably in the range of 0.2-0.4 bar, and/or that the time of treatment is in the range of 10-35 seconds, preferably in the range of 15-25 seconds, and/or that the distance from the jet to the implant is in the range of 30-50 mm, and/or the bore diameter of the jet is in the range of 0.8-1.2 mm.

8. Method according to one of the preceding claims, **characterized in that**, following the sintering, the porous surface is further modified at least area-wise with an erosive chemical or physical treatment.

9. Method according to claim 8, **characterized in that** the subsequent treatment comprises a molten salt modification carried out at least area-wise, **in that** the implant is structured by etching on the surface by a molten salt, wherein especially during etching essentially exclusively an erosion of material takes place.

10. Method according to claim 9, **characterized in that** the molten salt is a molten salt of alkali- and/or alkaline earth-nitrates, hydroxides or halogens, or a mixture of these salts.

11. Method according to one of the preceding claims 8 or 9, **characterized in that** the molten salt is a molten salt with at least one hydroxide, especially with at least one alkali- and/or alkaline earth-hydroxide, or that the molten salt is a molten salt consisting exclusively of one or more hydroxides, especially of one or more alkali- and/or alkaline earth-hydroxides
and/or **in that** the molten salt is a molten salt of potassium hydroxide, and/or sodium hydroxide, and/or lithium hydroxide..

12. Method according to one of the preceding claims 15 or 16, **characterized in that** the molten salt is a molten salt with at least one chloride, especially with at least one alkali- and/or alkaline earth chloride, or that the molten salt is a molten salt consisting exclusively of one or more chlorides, especially of one more alkali- and/or alkaline earth-chlorides wherein preferably the molten salt is a binary molten salt of potassium hydroxide and sodium hydroxide, or of potassium chloride and lithium chloride, in a ratio of 2:1 - 0.5:1, preferably in the range of 1.5:1 - 0.75:1, especially preferably in the range of 1:1 or 7:5, wherein preferably a temperature in the range of 100-600°C, especially in the range of 150-250°C is used, and/or **in that** the surface is exposed to a molten salt at least area-wise over a period of 10 minutes to 300 hours, preferably of at least 2 hours, preferably from 10-100 hours, especially from 25 to 35 hours.

13. Method according to one of the preceding claims, **characterized in that** the implant consists of ceramics, wherein it preferably contains zirconium oxide, to which possibly yttrium oxide and hafnium oxide is added, and/or that it contains aluminium oxide, to which possibly silicium dioxide, ferric (III) oxide, and/or sodium oxide is added, and/or that it contains silicium nitride, to which possibly silicium dioxide, ferric (III) oxide and/or sodium oxide is added, and/or that it contains titanium oxide, and/or that it is formed of mixtures of said materials.

## Revendications

1. Procédé de fabrication d'un implant métallique et/ou céramique présentant une surface structurée, en particulier poreuse, destiné à être inséré au moins en partie dans un tissu dur, par exemple dans un os et/ou dans un tissu osseux, au moins certaines parties de l'implant étant fabriquées en recourant à une compression isostatique à froid, un moulage et/ou une injection (CIM, MIM) pour former une ébauche qui est ensuite frittée pour fournir l'implant,
la surface étant modifiée et/ou préparée avant le frittage de telle sorte qu'après le frittage, on obtienne une surface macroporeuse et/ou macrostructurée,
l'ébauche étant modifiée après la compression isostatique à froid, la coulée et/ou l'injection et avant le frittage final en sablant la surface de l'ébauche,
l'agent utilisé pour le sablage étant un agent de sablage abrasif et/ou densifiant la surface, **caractérisé en ce que**
comme agent de sablage, le procédé utilise un mélange de deux agents de sablage différents qui présentent des granulométries différentes, la différence entre les tailles moyennes des grains de différents agents de sablage étant un facteur de l'ordre de 5 à 10.

2. Procédé selon la revendication 1, **caractérisé en ce qu'**après le frittage, l'ébauche présente sans traitement supplémentaire de finition une surface macroporeuse et/ou macrostructurée.

3. Procédé selon les revendications 1 ou 2, **caractérisé en ce que** l'agent de sablage abrasif et/ou densifiant la surface est un agent de sablage métallique, par exemple des billes d'acier, un agent de sablage céramique, par exemple en Al₂O₃, ZrO₂, SiO₂, phosphate de Ca, TiO₂, NaO₂, CaO, MgO, un agent de sablage organique ou naturel, par exemple des coques de noix ou du riz présentant différentes tailles de grains et de fragments ou des mélanges desdits agents de sablage, la taille des grains de l'agent de sablage étant de préférence comprise dans les plages de 0,01 à 0,25 mm et/ou de 0,01 à 0,2 mm, et/ou **en ce que** l'agent utilisé pour le sablage est un agent de sablage organique, par exemple des stéarates, des cires, des paraffines ou de préférence un carbamide, une résine de mélamine, du biuret, de la mélamine, du carbonate d'ammonium, du bicarbonate d'ammonium ou leurs mélanges.

4. Procédé selon la revendication 3, **caractérisé en ce qu'**avant le frittage qui suit, les agents de sablage peuvent être enlevés à une température d'au plus 600°C sans laisser de résidu, cet enlèvement étant de préférence réalisé dans une atmosphère oxydante, réductrice ou inerte, de préférence une atmosphère d'O₂, N₂, NH₄, Ar ou leurs mélanges, ou sous vide, le bicarbonate d'ammonium se sublimant de la surface de l'ébauche dès 65°C et laissant la structure souhaitée dans la surface.

5. Procédé selon l'une des revendications 3 et 4, **caractérisé en ce que** la pression de sablage est comprise dans la plage de 0,2 à 7 bars, de préférence entre 0,2 et 5 bars, de façon particulièrement préférable de l'ordre de 0,8 bar et/ou **en ce que** le traitement de sablage est réalisé pendant une durée comprise entre 15 et 65 secondes, de préférence entre 35 et 55 secondes et de façon particulièrement préférable de l'ordre de 50 secondes, la distance entre la tuyère et l'implant étant de préférence comprise entre 25 et 80 mm, en particulier entre 25 et 60 mm et de façon particulièrement préférable de l'ordre de 30 mm, pour un diamètre d'alésage de la tuyère compris dans la plage de 0,8 à 1,2 mm et de préférence dans la plage de 0,8 à 1,0 mm.

6. Procédé selon l'une des revendications 1 à 5, **caractérisé en ce que** le mélange présente un premier agent de sablage dont la taille moyenne des grains est comprise dans la plage de 0,1 à 0,2 mm, de préférence dans la plage de 0,2 à 0,8 mm, l'agent de sablage étant de préférence organique et en particulier constitué de noyaux de fruits, et **en ce que** le mélange présente un deuxième agent de sablage dont la taille moyenne des grains est comprise dans la plage de 0,01 à 0,1 mm et de préférence dans la plage de 0,03 à 0,9 mm, cet agent de sablage étant de préférence minéral et en particulier à base d'oxyde d'aluminium (Al₂O₃), le rapport entre le premier et le deuxième agent de sablage étant de préférence compris dans la plage de 5:1 à 1:5 et de préférence dans la plage de 3:1 à 1:1, le sablage s'effectuant de préférence en deux étapes, ledit mélange étant utilisé dans une première étape et seul l'agent de sablage en grains de la plus petite taille, de préférence un agent de sablage minéral, étant utilisé dans la deuxième étape, la deuxième étape étant conduite de préférence à une pression de sablage d'au moins 5 à 10 fois plus petite.

7. Procédé selon l'une des revendications 1 à 6, **caractérisé en ce que** l'étape qui utilise un mélange s'effectue à une pression comprise dans la plage de 2 à 7 bars et de préférence de 3 à 5 bars, **en ce que** la durée du traitement est comprise dans la plage de 15 à 65 secondes, de préférence dans la plage de 25 à 45 secondes, **en ce que** la distance entre la tuyère et l'implant est comprise dans la plage de 25 à 80 mm et/ou **en ce que** le diamètre de l'alésage de la tuyère est compris dans la plage de 1,2 à 2,0 mm, et dans le cas où une deuxième étape est prévue, elle utilise une pression dans la plage de 0,2 à 0,8 bar, de préférence dans la plage de 0,2 à 0,4 bar, la durée du traitement est comprise dans la plage de 10 à 35 secondes, de préférence dans la plage de 15 à 25 secondes, **en ce que** la distance entre la tuyère et l'implant est comprise dans la plage de 30 à 50 mm et/ou **en ce que** le diamètre de l'alésage de la tuyère est compris dans la plage de 0,8 mm à 1,2 mm.

8. Procédé selon l'une des revendications précédentes, **caractérisé en ce qu'**au moins certaines parties de la surface poreuse sont encore modifiées par un traitement d'abrasion chimique ou physique qui suit le frittage.

9. Procédé selon la revendication 14, **caractérisé en ce que** le traitement suivant comporte une modification dans un sel fondu qui a lieu au moins dans certaines parties en structurant la surface de l'implant par gravure par un bain de sel fondu, essentiellement uniquement un enlèvement de matière ayant lieu lors de la gravure dans le bain de sel fondu.

10. Procédé selon la revendication 15, **caractérisé en ce que** le bain de sel fondu est un bain de sel fondu de nitrate, d'hydroxyde ou d'halogénure de métal alcalin ou de métal alcalino-terreux ou un mélange de ces sels.

11. Procédé selon l'une des revendications 15 ou 16 qui précèdent, **caractérisé en ce que** le bain de sel fondu est un bain de sel fondu qui présente au moins un hydroxyde, en particulier au moins un hydroxyde de métal alcalin et/ou de métal alcalino-terreux, **en ce que** le bain de sel fondu est un bain de sel fondu constitué exclusivement d'un ou de plusieurs hydroxydes, en particulier d'un ou de plusieurs hydroxydes de métal alcalin et/ou de métal alcalino-terreux et/ou **en ce que** le bain de sel fondu est un bain de sel fondu d'hydroxyde de potassium, d'hydroxyde de sodium et/ou d'hydroxyde de lithium.

12. Procédé selon l'une des revendications 15 ou 16 qui précèdent, **caractérisé en ce que** le bain de sel fondu est un bain de sel fondu qui présente au moins un chlorure, en particulier au moins un chlorure de métal alcalin et/ou de métal alcalino-terreux, **en ce que** le bain de sel fondu est un bain de sel fondu constitué exclusivement d'un ou de plusieurs chlorures, en particulier d'un ou de plusieurs chlorures de métal alcalin et/ou de métal alcalino-terreux et/ou **en ce que** le bain de sel fondu est un bain de sel fondu de chlorure de potassium, de chlorure de sodium et/ou de chlorure de lithium, dans un rapport compris dans la plage de 2,1 à 0,5:1, de préférence dans la plage de 1,5:1 à 0,75:1, de façon particulièrement préférable dans la plage de 1:1 ou de 7:5, de préférence à une température comprise dans la plage de 100 à 600°C et en particulier dans la plage de 150 à 250°C, au moins certaines parties de la surface étant exposées à un bain de sel fondu pendant une durée de 10 minutes à 300 heures, de préférence d'au moins 2 heures et de manière préférable de 10 à 100 heures, en particulier de 25 à 35 heures.

13. Procédé selon l'une des revendications précédentes, **caractérisé en ce que** l'implant est constitué de céramique, cet implant contenant de préférence de l'oxyde de zirconium auquel sont éventuellement ajoutés de l'oxyde d'yttrium et de l'oxyde de hafnium, **en ce qu'**il contient de l'oxyde d'aluminium auquel sont éventuellement ajoutés du dioxyde de silicium, de l'oxyde de fer(III) et/ou de l'oxyde de sodium, **en ce qu'**il contient du nitrure de silicium auquel sont éventuellement ajoutés du dioxyde de silicium, de l'oxyde de fer(III) et/ou de l'oxyde de sodium, **en ce qu'**il contient de l'oxyde de titane et/ou en ce qu'il est formé de mélanges desdits matériaux.
